(19) Europäisches Patentamt
European Patent Office
Office européen des brevets

(11) **EP 4 538 702 A1**

(12) **EUROPEAN PATENT APPLICATION**

(43) Date of publication:
16.04.2025 Bulletin 2025/16

(21) Application number: 24205823.8

(22) Date of filing: 10.10.2024

(51) International Patent Classification (IPC):
*G01N 33/18* (2006.01)     *C12Q 1/02* (2006.01)
*C12Q 1/22* (2006.01)     *G01N 21/76* (2006.01)

(52) Cooperative Patent Classification (CPC):
G01N 33/1866; C12Q 1/02; C12Q 1/22;
G01N 21/763; G01N 33/1826

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 13.10.2023 KR 20230136837

(71) Applicants:
• **Korea Institute of Ocean Science & Technology Busan 49111 (KR)**
• **Dongmoonent Co., Ltd Seoul 08377 (KR)**

(72) Inventors:
• **LEE, Kyoung Jin**
  **08324 Seoul (KR)**
• **LEE, Dong Kwon**
  **16855 Yongin-si, Gyeonggi-do (KR)**
• **KIM, Jong Young**
  **14537 Bucheon-si, Gyeonggi-do (KR)**
• **PARK, Chul Woo**
  **04311 Seoul (KR)**
• **LEE, Moon Jin**
  **34033 Daejeon (KR)**
• **CHOI, Hoon**
  **22374 Incheon (KR)**

(74) Representative: **Beck & Rössig**
**European Patent Attorneys**
**Denninger Str. 169**
**81925 München (DE)**

(54) **ECOTOXICITY MONITORING DEVICE USING ALIIVIBRIO FISCHERI AND ECOTOXICITY MONITORING METHOD USING THE SAME**

(57)     One embodiment of the present invention provides an ecotoxicity monitoring device using *Aliivibrio fischeri,* and an ecotoxicity monitoring method using the same. The ecotoxicity monitoring device includes a reaction tank module including a reference reaction tank, a sample reaction tank, and a dilution reaction tank and provided with a bacterial dilution tank configured to supply a mixed solution including *Aliivibrio fischeri* to the reference reaction tank, the sample reaction tank, and the dilution reaction tank; an *Aliivibrio fischeri* culture unit configured to supply *Aliivibrio fischeri,* which detect whether a sample is toxic, to the bacterial dilution tank; a diluted water supply unit configured to supply diluted water to the bacterial dilution tank, the reference reaction tank, and the dilution reaction tank; a sample supply unit configured to supply a sample to the sample reaction tank and the dilution reaction tank; an optical measurement unit configured to measure an amount of luminescence of *Aliivibrio fischeri* in a toxicity assessment substance provided from the reference reaction tank and the sample reaction tank and the dilution reaction tank to which the sample is supplied; a control unit configured to control the operation of the reaction tank module, the *Aliivibrio fischeri* culture unit, the diluted water supply unit, the sample supply unit, and the optical measurement unit; and an analysis unit configured to judge whether a toxic substance is present in the sample based on the light quantity value provided from the optical measurement unit, wherein the analysis unit is configured to calculate a correction coefficient based on the initial light quantity value of the reference reaction tank measured through the optical measurement unit and the final light quantity value measured after a predetermined time has elapsed, and calculate the corrected light quantity values of the sample reaction tank and the dilution reaction tank based on the calculated correction coefficient.

EP 4 538 702 A1

1000

510    510    510    500

110    300

120    130    140    400

← — — MOVING FLOW OF DILUTED WATER
← · — — MOVING FLOW OF SAMPLE

**FIG. 4**

## Description

CROSS-REFERENCE TO RELATED APPLICATION

This application claims priority to and the benefit of Korean Patent Application No. 10-2023-0136837, filed on October 13, 2023, the disclosure of which is incorporated herein by reference in its entirety.

BACKGROUND

### 1. Field of the Invention

The present invention relates to an ecotoxicity monitoring device using *Aliivibrio fischeri,* and an ecotoxicity monitoring method using the same, and more particularly, to an ecotoxicity monitoring device using *Aliivibrio fischeri,* which is configured to rapidly and accurately monitor whether toxic substances are present in a sample, and an ecotoxicity monitoring method using the same.

### 2. Discussion of Related Art

Facilities for treating sewage and wastewater are being installed and operated to improve water quality and protect the ecosystem, but there is a problem that hazardous chemicals cannot be completely removed through biological or physicochemical treatment processes. As a result, the hazardous chemicals that are not removed during the sewage and wastewater treatment process are discharged into public waters and cause adverse effects on the ecosystem and health.

Also, hazardous noxious substances (HNSs) that are discharged into the ocean by marine industrial facilities or ship accidents are causing direct and indirect damage to the surrounding ocean and seabed, ocean users, coastal residents, and the like. These HNSs discharged into the ocean are diverse, and information on the types and characteristics of substances through physicochemical analysis alone without preliminary information is limited. In addition, it is realistically impossible for physicochemical analysis technology to analyze toxic substances for all samples in terms of cost, time, technology, and human resources required for the analysis.

Therefore, ecotoxicity assessment/analysis technology has emerged as a scientific and efficient management method for environmental analysis. This ecotoxicity assessment/analysis technology is a technology that uses living organisms to evaluate the harmfulness of substances existing in the environment.

However, conventional ecotoxicity assessment devices have a problem in that even the fastest ones require at least 15 minutes of assessment time to evaluate whether there are toxic substances in in a sample. Therefore, when it is evaluated whether there are toxic substances in a sample using an ecotoxicity assessment device, there is a problem in that the longer assessment time makes it more difficult to respond quickly to environmental pollution, which results in increased damage.

Accordingly, various research and development are being conducted on ecotoxicity monitoring devices that can more quickly and accurately monitor HNSs flowing into rivers and oceans.

[Related-Art Document]

[Patent Document]

Patent Document 1: Korean Publication Patent No. 2014-0093389 (July 28, 2014)

SUMMARY OF THE INVENTION

The present invention is designed to solve the above problems, and therefore it is an object of the present invention to provide an ecotoxicity monitoring device using *Aliivibrio fischeri,* which is configured to rapidly and accurately monitor whether toxic substances are present in a sample, and an ecotoxicity monitoring method using the same.

According to an aspect of the present invention, there is provided an ecotoxicity monitoring device using *Aliivibrio fischeri,* which includes a reaction tank module including a reference reaction tank, a sample reaction tank, and a dilution reaction tank and provided with a bacterial dilution tank configured to supply a mixed solution including *Aliivibrio fischeri* to the reference reaction tank, the sample reaction tank, and the dilution reaction tank; an *Aliivibrio fischeri* culture unit configured to supply *Aliivibrio fischeri,* which detect whether a sample is toxic, to the bacterial dilution tank; a diluted water supply unit configured to supply diluted water to the bacterial dilution tank, the reference reaction tank, and the dilution reaction tank; a sample supply unit configured to supply a sample to the sample reaction tank and the dilution reaction tank; an optical measurement unit configured to measure an amount of luminescence of *Aliivibrio fischeri* in a toxicity assessment substance provided from the reference reaction tank and the sample reaction tank and the dilution reaction tank to which the sample is supplied; a control unit configured to control the operation of the reaction tank module, the *Aliivibrio fischeri* culture unit, the diluted water supply unit, the sample supply unit, and the optical measurement unit; and an analysis unit configured to judge whether toxic substances are present in the sample based on the light quantity value provided from the optical measurement unit, wherein the analysis unit is configured to calculate a correction coefficient based on the initial light quantity value of the reference reaction tank measured through the optical measurement unit and the final light quantity value measured after a predetermined time has elapsed, and calculate the corrected light quantity values of the sample reaction tank and the dilution reaction tank based on the calculated correction coefficient.

According to one embodiment of the present invention, the analysis unit may calculate the toxicity index (TIs) of the sample reaction tank, wherein the toxicity index (TIs) of the sample reaction tank may be calculated by calculating the correction coefficient of the reference reaction tank according to the following Calculation Formula 1, calculating a corrected light quantity value of the sample reaction tank to which the correction coefficient is applied according to the following Calculation Formula 2, and calculating the toxicity index (TIs) based on the final light quantity value of the sample reaction tank measured after a desired sample has been injected from the sample supply unit into the sample reaction tank and a predetermined time has elapsed according to the following Calculation Formula 3:

[Calculation Formula 1]

$$C0 = \left| \frac{R1}{R0} \right|$$

[R0 = Initial light intensity value of mixed solution supplied from bacterial dilution tank to reference reaction tank after 1 minute, and R1 = Final light quantity value after diluted water has been quantitatively injected into mixed solution in reference reaction tank and predetermined time has elapsed]

[Calculation Formula 2]

$$S1 = |S0 \times C0|$$

[S0 = Initial light intensity value of mixed solution supplied from bacterial dilution tank to sample reaction tank after 1 minute, and C0 = Correction coefficient]

[Calculation Formula 3]

$$TI_S = \left[ \frac{(S1 - S2)}{S1} \right] \times 100$$

[S1 = Corrected light quantity value after initial 1 minute in sample reaction tank to which correction coefficient is applied, and S2 = Final light quantity value after sample is quantitatively injected into mixed solution in sample reaction tank and predetermined time has elapsed].

According to one embodiment of the present invention, the analysis unit may calculate the toxicity index ($TI_d$) of the dilution reaction tank, wherein the toxicity index ($TI_d$) of the dilution reaction tank may be calculated by calculating a corrected light quantity value of the dilution reaction tank to which the correction coefficient of the reference reaction tank is applied according to the following Calculation Formula 4, and calculating the toxicity index ($TI_d$) based on the final light quantity value of the dilution reaction tank measured after the sample and diluted water have been injected from the sample supply unit and the diluted water supply unit into the dilution reaction tank at the desired ratio and a predetermined time has elapsed according to the following Calculation Formula 5:

[Calculation Formula 4]

$$D1 = |D0 \times C0|$$

[D0 = Initial light intensity value of mixed solution supplied from bacterial dilution tank to dilution reaction tank after 1 minute, and C0 = Correction coefficient]

[Calculation Formula 5]

$$TI_d = \left[ \frac{(D1 - D2)}{D1} \right] \times 100$$

[D1 = Corrected light intensity value of dilution reaction tank to which correction coefficient is applied after initial 1 minute, and D2 = Final light quantity value after sample and diluted water have been quantitatively injected into mixed solution in dilution reaction tank and predetermined time has elapsed].

According to one embodiment of the present invention, the analysis unit may judge that the calculated correction coefficient is valid if it ranges from 0.6 to 1.3, and judge that a toxic substance is present in the sample if the calculated toxicity index (TIs) of the sample reaction tank ranges from 20 to 80%.

According to one embodiment of the present invention, the analysis unit may evaluate the toxicity risk of the sample by comparing the toxicity index (TIs) of the sample reaction tank and the toxicity index ($TI_d$) of the dilution reaction tank.

According to one embodiment of the present invention, the predetermined time taken to measure the final light quantity values of the reference reaction tank, the sample reaction tank, and the dilution reaction tank may be 5 minutes.

According to another aspect of the present invention, there is provided an ecotoxicity monitoring method using *Aliivibrio fischeri,* which includes: (A) preparing a mixed solution in which *Aliivibrio fischeri* and diluted water are mixed in a bacterial dilution tank; (B) supplying the mixed solution in the bacterial dilution tank to each of a reference reaction tank, a sample reaction tank and a dilution reaction tank; (C) measuring the initial light quantity value of *Aliivibrio fischeri* in the reference reaction tank, the sample reaction tank, and the dilution reaction tank through an optical measurement unit after a predetermined time has elapsed; (D) supplying diluted water to the reference reaction tank, supplying the sample to the sample reaction tank, and supplying predetermined amounts of the sample and diluted water to the dilution reaction tank after the initial light quantity value measurement; (E) measuring the final light quantity value of *Aliivibrio fischeri* in the reference reaction tank, the sample reaction tank, and the dilution reaction tank through the optical measurement unit after a predetermined time has elapsed; and (F) providing the initial light quantity values and the final light quantity values of the respective reaction tanks measured through the optical measurement unit to the analysis unit to judge whether a toxic substance is present in the sample at the analysis unit, wherein the analysis unit is configured to calculate a toxicity index for the toxicity assessment substance in the sample reaction tank and the dilution reaction tank by calculating a correction coefficient based on the initial light quantity value and the final light quantity value of the reference reaction tank measured through the optical measurement unit, and calculating the corrected light quantity values of the sample reaction tank and the dilution reaction tank based on the calculated correction coefficient.

## BRIEF DESCRIPTION OF THE DRAWINGS

FIG. 1 is a block diagram of an ecotoxicity monitoring device using *Aliivibrio fischeri* according to one embodiment of the present invention.
FIG. 2 is an exemplary diagram of the ecotoxicity monitoring device using *Aliivibrio fischeri* according to one embodiment of the present invention.
FIG. 3 is an exemplary diagram schematically showing a process of supplying a mixed solution accommodated in a bacterial dilution tank according to one embodiment of the present invention to a reference reaction tank, a sample reaction tank, and a dilution reaction tank, and then measuring an initial light quantity value.
FIG. 4 is an exemplary diagram schematically showing a process of supplying a desired sample and diluted water to a reference reaction tank, a sample reaction tank, and a dilution reaction tank according to one embodiment of the present invention, and then measuring a final light quantity value.
FIG. 5 is a flowchart showing an ecotoxicity monitoring method using *Aliivibrio fischeri* according to one embodiment of the present invention.

## DETAILED DESCRIPTION OF EXEMPLARY EMBODIMENTS

Hereinafter, the present invention will be described with reference to the accompanying drawings.

However, it should be understood that the present invention can be implemented in various other forms and is not intended to be limited to the exemplary embodiments of the present invention. Also, in the drawings, descriptions of parts unrelated to the detailed description will be omitted to clearly describe one aspect of the present invention. Throughout the specification, like numbers refer to like elements.

Throughout the specification, when a certain element is referred to as being "connected" to another element, it can be "directly connected" to the other element or "indirectly connected" to the other element with one or more intervening elements interposed therebetween. In addition, when an element is referred to as "including" another element, it should be understood that the element may further include still another element rather than excluding other elements unless particularly indicated otherwise.

In the present invention, the terms "upper" and "lower" mean that an object is located above or below the target member, but do not necessarily mean that the object is located above or below with respect to the direction of gravity.

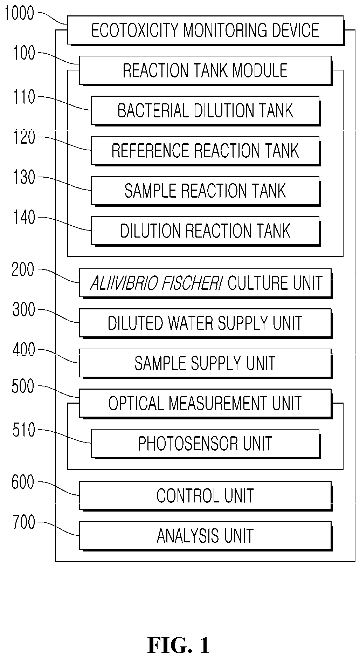

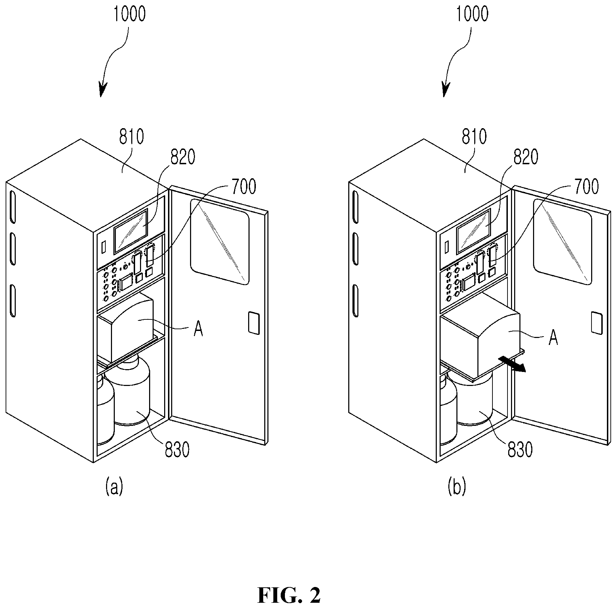

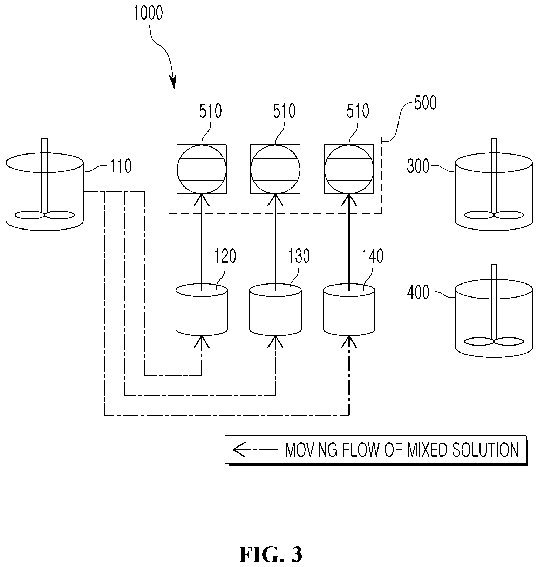

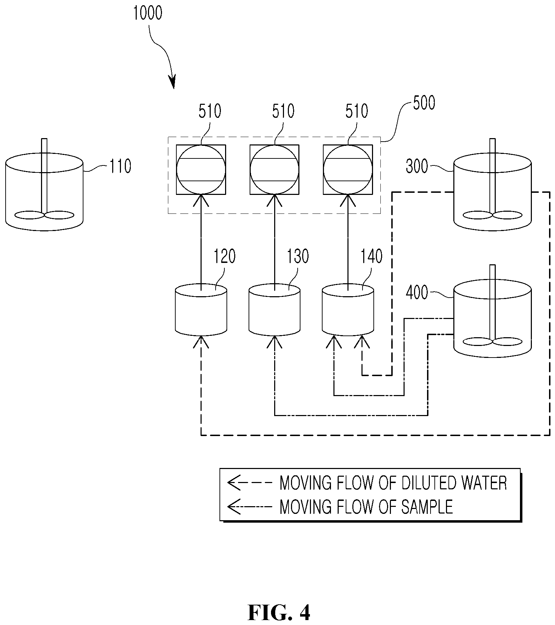

Hereinafter, embodiments of the present invention will be described in detail with reference to the accompanying drawings. FIG. 1 is a block diagram of an ecotoxicity monitoring device using *Aliivibrio fischeri* according to one embodiment of the present invention, FIG. 2 is an exemplary diagram of the ecotoxicity monitoring device using *Aliivibrio fischeri* according to one embodiment of the present invention, FIG. 3 is an exemplary diagram schematically showing a process of supplying a mixed solution accommodated in a bacterial dilution tank according to one embodiment of the present invention to a reference reaction tank, a sample reaction tank, and a dilution reaction tank, and then measuring an initial light quantity value, and FIG. 4 is an exemplary diagram schematically showing a process of supplying a desired sample and diluted water to a reference reaction tank, a sample reaction tank, and a dilution reaction tank according to one embodiment of the present invention, and then measuring a final light quantity value.

As shown in FIGS. 1 to 4, an ecotoxicity monitoring device 1000 using *Aliivibrio fischeri* may include a reaction tank module 100, an *Aliivibrio fischeri* culture unit 200, a diluted water supply unit 300, a sample supply unit 400, an optical measurement unit 500, a control unit 600, and an analysis unit 700.

Such an ecotoxicity monitoring device 1000 may, for example, be provided with a casing 810 configured to protect various apparatuses installed inside from the outside.

The interior of such a casing 810 may be partitioned. For example, a monitor unit 820 configured to present the toxicity measurement results may be provided on the upper side of the casing 810, the analysis unit 700 may be provided on the lower side of the monitor unit 820, an autosampler A may be provided on the lower side of the analysis unit 700, and a reagent, a waste liquid storage unit 830, and the like may be stored on the lower side of the autosampler A. The arrangement of respective components provided in such a casing 810 does not necessarily have to be configured in the form described above, and may be provided in various forms other than that described above.

Here, the autosampler A provided with the reaction tank module 100 and the optical measurement unit 500 provided in the ecotoxicity monitoring device 1000 may, for example, be easily withdrawn to the outside in a sliding mode, or may be introduced into the casing 810 from the outside. In this way, when a problem occurs in one of multiple components provided in the autosampler A that is configured to be slidable, a user may easily repair or replace the corresponding component that has failed while the autosampler A itself is withdrawn to the outside.

Such an ecotoxicity monitoring device 1000 is configured to judge whether a toxic substance is present in a sample using *Aliivibrio fischeri*. That is, the ecotoxicity monitoring device 1000 judges whether toxic substances are present in a sample by measuring the rate of change in an amount of luminescence of *Aliivibrio fischeri* according to the input of the sample. In this way, although determining whether toxic substances are present in the sample based on the rate of change in the amount of luminescence of *Aliivibrio fischeri* has already been disclosed in the prior art, the analysis unit 700 provided in the ecotoxicity monitoring device 1000 of the present invention is configured to calculate a correction coefficient based on the initial and final light quantity values measured from the reference reaction tank 120, and more quickly judge whether toxic substances are present in the toxicity assessment substance in the sample reaction tank 130 and the dilution reaction tank 140 based on the calculated correction coefficient.

Specifically, the reaction tank module 100 may include a bacterial dilution tank 110, a reference reaction tank 120, a sample reaction tank 130, and a dilution reaction tank 140.

Here, an accommodation space is provided in the bacterial dilution tank 110 so that a mixed solution including *Aliivibriofischeri* and diluted water can be accommodated into the bacterial dilution tank 110. This bacterial dilution tank 110 receives *Aliivibrio fischeri* from the *Aliivibrio fischeri* culture unit 200, and receives diluted water from the diluted water supply unit 300.

Here, *Aliivibrio fischeri* that detects whether a sample is toxic is cultured in the *Aliivibrio fischeri* culture unit 200. Such an *Aliivibrio fischeri* culture unit 200 may be configured to maintain a temperature of approximately 4 °C, for example, for the purpose of growth inhibition.

Also, the diluted water supply unit 300 is, for example, configured to store diluted water, that is, mineral water. The diluted water stored in such a diluted water supply unit 300 does not necessarily have to be made of only mineral water, and saline (NaCl) and the like may also be stored.

In this way, the mixed solution including *Aliivibrio fischeri* and diluted water supplied to the bacterial dilution tank 110 may be uniformly mixed through a stirrer provided in the bacterial dilution tank 110.

Such a bacterial dilution tank 110 is configured to send a mixed solution in which *Aliivibrio fischeri* and diluted water are mixed at a predetermined ratio to each of the reference reaction tank 120, the sample reaction tank 130, and the dilution reaction tank 140. At this time, the mixed solution sent from the bacterial dilution tank 110 to each of the reference reaction tank 120, the sample reaction tank 130, and the dilution reaction tank 140 may be sent via, for example, a transfer pump (not shown). The movement of the sample and/or diluted water (including such a mixed solution) as will be described later may be performed by the operation of each transfer pump connected to the sample supply unit 400 and/or the diluted water supply unit 300.

Also, for the mixed solution sent to the reference reaction tank 120, the sample reaction tank 130, and the dilution reaction tank 140, the initial light quantity value of *Aliivibriofischeri* in each reaction tank is measured through the optical measurement unit 500 after a predetermined time (1 minute) has elapsed.

Such an optical measurement unit 500 is provided with a photosensor unit 510. In this case, the photosensor unit 510 is configured to measure the amount of luminescence from *Aliivibrio fischeri* that has entered the internal cell and provide the measured initial light quantity value to the analysis unit 700.

Here, the cell may be made of a quartz material to optimally detect the quantity of light. In addition, the cell may be formed as a dark room to detect the emitted light. The photosensor unit 510 may be, for example, a photo multiplier tube (PMT). This photosensor unit 510 measures the initial amount of luminescence of *Aliivibrio fischeri* when no sample is added and the final amount of luminescence of *Aliivibriofischeri* after the sample has been added and a predetermined time has elapsed.

These photosensor units 510 are provided in a number corresponding to the plurality of reaction tanks. That is, the photosensor units 510 may be provided in a number corresponding to the number of the reference reaction tanks 120, the sample reaction tanks 130, and the dilution reaction tanks 140.

Here, the reference reaction tank 120, the sample reaction tank 130, and the dilution reaction tank 140 communicate with each of the photosensor units 510. Accordingly, the mixed solution including *Aliivibrio fischeri* accommodated in the reference reaction tank 120, the sample reaction tank 130, and the dilution reaction tank 140 may be supplied to each of the photosensor units 510. Because each of such photosensor units 510 communicates with the reference reaction tank 120, the sample reaction tank 130, and the dilution reaction tank 140, the mixed solution accommodated in the cell of the photosensor unit 510 in which the light quantity measurement is completed may be discharged to each of the reaction tanks.

In this way, the light quantity value of each of the reaction tanks measured from the photosensor unit 510 is provided to the analysis unit 700.

After the light quantity measurement for the mixed solution is completed, the cell inside such a photosensor unit 510 may be diluted with the diluted water provided from the diluted water supply unit 300. That is, the photosensor unit 510 is configured to be washed prior to a new light quantity measurement. Accordingly, the photosensor unit 510 may measure an accurate light quantity value for the provided assessment substance without being affected by previously used samples or *Aliivibriofischeri.*

In this way, when the measurement of the initial light quantity value of the mixed solution supplied from the reference reaction tank 120, the sample reaction tank 130, and the dilution reaction tank 140 is completed, a predetermined amount of diluted water is additionally supplied to the reference reaction tank 120, a sample is supplied to the sample reaction tank 130, and the sample and diluted water are supplied to the dilution reaction tank 140 at a certain ratio. Here, the sample supply unit 400 supplies the sample only to the sample reaction tank 130 and the dilution reaction tank 140. In this way, the assessment substance in the sample reaction tank 130 and the dilution reaction tank 140 to which the sample is additionally supplied may be a toxicity assessment substance.

In this way, when a predetermined amount of the sample and/or diluted water is additionally supplied to the reference reaction tank 120, the sample reaction tank 130, and the dilution reaction tank 140, the final light intensity values for the assessment substance in the reference reaction tank 120 to which only diluted water is added, and the toxicity assessment substance in the sample reaction tank 130 and the dilution reaction tank 140 are measured after a certain time (5 minutes) has elapsed.

In this way, each of the final light quantity values measured from the photosensor unit 510 is provided to the analysis unit 700. Then, the assessment substance/toxicity assessment substance accommodated in the photosensor unit 510 is discharged to the corresponding reaction tank, and the cell inside the photosensor unit 510 is washed with diluted water.

Then, after the final light quantity value of each reaction tank is measured, the assessment substance/toxicity assessment substance accommodated in the reference reaction tank 120, the sample reaction tank 130, and the dilution reaction tank 140 is discharged to the waste liquid storage unit 830 through a transfer pump. Then, the reference reaction tank 120, the sample reaction tank 130, and the dilution reaction tank 140 are washed with diluted water for the next assessment test.

Meanwhile, the control unit 600 controls the operation of various components provided in the ecotoxicity monitoring device 1000, such as the reaction tank module 100, the *Aliivibrio fischeri* culture unit 200, the diluted water supply unit 300, the sample supply unit 400, the optical measurement unit 500, and the like. For example, such a control unit 600 controls various tasks, such as supplying a sample and/or diluted water to the reaction tank module 100 or washing the photosensor unit 510.

Meanwhile, the analysis unit 700 is configured to calculate a toxicity index based on each of the initial and final light quantity values measured for the assessment substance/toxicity assessment substance provided to the photosensor unit 510 from the aforementioned reference reaction tank 120, sample reaction tank 130, and dilution reaction tank 140.

Such an analysis unit 700 is configured to calculate the toxicity index (TIs) of the sample reaction tank 130.

This toxicity index (TIs) of the sample reaction tank is calculated by calculating the correction coefficient of the reference reaction tank 120 according to the following Calculation Formula 1:

[Calculation Formula 1]

$$C0 = \left| \frac{R1}{R0} \right|$$

[R0 = Initial light intensity value of mixed solution supplied from bacterial dilution tank to reference reaction tank after 1 minute, and R1 = Final light quantity value after diluted water has been quantitatively injected into mixed solution in reference reaction tank and 5 minutes have elapsed]

At this time, the analysis unit 700 judges that the calculated correction coefficient is valid if it ranges from 0.6 to 1.3, and conducts a toxicity assessment test again when a fresh mixed solution is supplied from the bacterial dilution tank 110 to each reaction tank if the correction coefficient falls outside the range of 0.6 to 1.3.

Then, the analysis unit 700 calculates the corrected light quantity value of the sample reaction tank 130 to which the correction coefficient is applied according to the following Calculation Formula 2. Here, the corrected light quantity value may be the light quantity value of *Aliivibriofischeri* to which the natural occurrence or reduction rate of *Aliivibrio fischeri* is applied:

[Calculation Formula 2]

$$S1 = |S0 \times C0|$$

[S0 = Initial light intensity value of mixed solution supplied from bacterial dilution tank to sample reaction tank after 1 minute, and C0 = Correction coefficient]

Also, the analysis unit 700 calculates the toxicity index (TIs) based on the final light quantity value of the sample reaction tank 130 measured after a predetermined desired has been is injected into the sample reaction tank 130 from the sample supply unit 400 and a predetermined time (5 minutes) has elapsed according to the following Calculation Formula 3:

[Calculation Formula 3]

$$TI_S = \left[\frac{(S1 - S2)}{S1}\right] \times 100$$

[S 1 = Corrected light quantity value after initial 1 minute in sample reaction tank to which correction coefficient is applied, and S2 = Final light quantity value after sample has been quantitatively injected into mixed solution in sample reaction tank and predetermined time has elapsed]

In this way, when the toxicity index (TIs) of the sample reaction tank 130 calculated through the analysis unit 700 ranges from 20 to 80%, the analysis unit 700 judges that a toxic substance is present in the sample. This analysis unit 700 calculates a correction coefficient based on the initial light intensity value of the reference reaction tank 120 and the final light intensity value measured after a predetermined time (5 minutes) has elapsed, and quickly judges whether a toxic substance is present in the sample accommodated in the sample reaction tank 130 based on the calculated correction coefficient. In this way, the ecotoxicity monitoring device 1000 according to the present invention may judge whether toxic substances are present in the sample more quickly than conventional ecotoxicity monitoring devices using luminescent bacteria.

Meanwhile, the analysis unit 700 calculates the toxicity index $(TI_d)$ of the dilution reaction tank 140 in the same manner as the sample reaction tank 130.

To calculate such a toxicity index $(TI_d)$ of the dilution reaction tank 140, the correction coefficient of the reference reaction tank 120 described above is used. The details of the correction coefficient of the reference reaction tank 120 have been described above, and thus the description thereof is omitted here.

Then, the analysis unit 700 calculates the corrected light quantity value of the dilution reaction tank 140 to which the correction coefficient is applied according to the following Calculation Formula 4. Here, the corrected light quantity value may be the light quantity value of *Aliivibriofischeri* to which the natural occurrence or reduction rate of *Aliivibrio fischeri* is applied:

[Calculation Formula 4]

$$D1 = |D0 \times C0|$$

[D0 = Initial light intensity value of mixed solution supplied from bacterial dilution tank to dilution reaction tank after 1 minute, and C0 = Correction coefficient]

Also, the analysis unit 700 injects the sample and diluted water from the sample supply unit 400 and the diluted water supply unit 300 into the dilution reaction tank 140 at the desired ratio according to the following Calculation Formula 5, and calculates the toxicity index $(TI_d)$ based on the final light quantity value of the dilution reaction tank 140 measured after a predetermined time (5 minutes) has elapsed. Here, the ratio of the sample and diluted water injected into the dilution reaction tank 140 may be 1: 1:

[Calculation Formula 5]

$$TI_d = \left[\frac{(D1 - D2)}{D1}\right] \times 100$$

[D 1 = Corrected light intensity value of dilution reaction tank to which correction coefficient is applied after initial 1 minute, and D2 = Final light quantity value after sample and diluted water have been quantitatively injected into mixed solution in dilution reaction tank and predetermined time has elapsed]

In this way, the toxicity index $(TI_d)$ of the dilution reaction tank 140 calculated through the analysis unit 700 is used to evaluate the toxicity risk of the sample. For example, when the toxicity index (TIs) of the sample reaction tank 130 calculated through the analysis unit 700 is 50%, the toxicity index $(TI_d)$ of the dilution reaction tank 140 should be approximately 25%. However, when the toxicity index (TIs) of the sample reaction tank 130 calculated through the analysis unit 700 is 50%, and the toxicity index $(TI_d)$ of the dilution reaction tank 140 is 40%, it may be judged that a large number of *Aliivibrio fischeri* were killed even in a small amount of the sample, indicating that the toxicity risk of the sample is very high.

In this way, the analysis unit 700 evaluates the toxicity risk of the sample by comparing the toxicity index (TIs) of the sample reaction tank 130 and the toxicity index $(TI_d)$ of the dilution reaction tank 140.

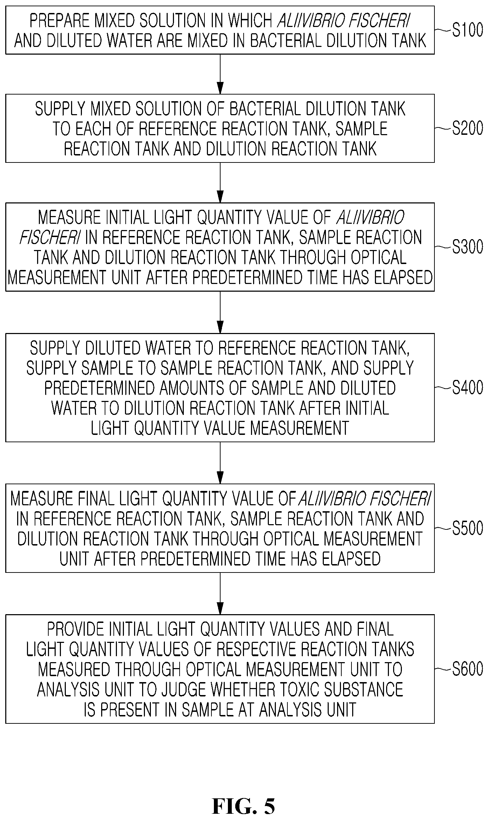

FIG. 5 is a flowchart showing the ecotoxicity monitoring method using *Aliivibrio fischeri* according to one embodiment of the present invention.

Referring schematically to the ecotoxicity monitoring method shown in FIG. 5, first, a mixed solution in which *Aliivibrio fischeri* and diluted water are mixed in a bacterial dilution tank 110 is prepared. This bacterial dilution tank 110 receives *Aliivibrio fischeri* from an *Aliivibrio fischeri* culture unit 200, and receives diluted water from a diluted water supply unit 300 (S100).

Next, the mixed solution in the bacterial dilution tank 110 is supplied to each of the reference reaction tank 120, the sample reaction tank 130, and the dilution reaction tank 140 (S200).

Then, after a predetermined time has elapsed, the initial light quantity value of *Aliivibriofischeri* in each of the reference reaction tank 120, the sample reaction tank

130, and the dilution reaction tank 140 is measured through the optical measurement unit 500. Here, the predetermined time taken for the optical measurement unit 500 to measure the initial light quantity values of *Aliivibrio fischeri* in the reference reaction tank 120, the sample reaction tank 130, and the dilution reaction tank 140 may be 1 minute. In this way, the initial light quantity values measured from the respective reaction tanks are provided to the analysis unit 700 (S300).

Subsequently, after the initial light quantity value measurement, diluted water is supplied to the reference reaction tank 120, the sample is supplied to the sample reaction tank 130, and predetermined amounts of the sample and diluted water are supplied to the dilution reaction tank 140. These additional diluted water and sample supplied to the reference reaction tank 120, the sample reaction tank 130, and the dilution reaction tank 140 may be supplied from the diluted water supply unit 300 and the sample supply unit 400 (S400).

Then, after a predetermined time has elapsed, the final light quantity values of *Aliivibrio fischeri* in the reference reaction tank 120, the sample reaction tank 130, and the dilution reaction tank 140 are measured through the optical measurement unit 500. Here, the predetermined time may be 5 minutes. In this way, the final light quantity values measured from the respective reaction tanks are provided to the analysis unit 700 (S500).

Finally, the analysis unit 700 calculates a toxicity index using the initial and final light quantity values of each reaction tank measured through the optical measurement unit 500, and determines whether toxic substances are present in the sample using the calculated toxicity index (S600).

Here, the analysis unit 700 calculates a correction coefficient based on the initial and final light quantity values of the reference reaction tank 120 measured through the optical measurement unit 500, and calculates the corrected light quantity values of the sample reaction tank 130 and the dilution reaction tank 140 based on the calculated correction coefficient, thereby finally calculating the toxicity indexes of the sample reaction tank 130 and the dilution reaction tank 140.

The effects of the above-described ecotoxicity monitoring device using *Aliivibrio fischeri* and ecotoxicity monitoring method using the same, which are configured to rapidly and accurately monitor whether toxic substances are present in a sample according to the present invention, are as follows.

According to the present invention, the analysis unit can calculate a correction coefficient based on the initial and final light quantity values measured from the reference reaction tank, and quickly calculate a toxicity index for the toxicity assessment substance in the sample reaction tank and the dilution reaction tank based on the calculated correction coefficient. Because such an ecotoxicity monitoring device using *Aliivibrio fischeri* can judge whether toxic substances are present in a sample within 5 minutes, it is possible to quickly respond to hazardous and noxious substances discharged into rivers or oceans.

It should be understood that the effects of the present invention are not limited to the effects described above and include all effects that can be deduced from the detailed description of the present invention or the configuration of the invention described in the claims.

However, this is only one preferred embodiment of the present invention, and the scope of the present invention is not limited by the scope described in this embodiment.

The foregoing description of the present invention is intended for illustration, and it will be understood by those skilled in the art to which the invention pertains that the invention can be easily modified into other specific forms. Therefore, it should be understood that the embodiments described above are only exemplary in all aspects and not limiting. For example, each of the components described as being one combined entity may be implemented separately, and similarly, components described as being separate entities may be implemented in a combined form.

It should be understood that the scope of the specification is defined by the following claims and that all changes or modifications derived from the meaning and scope of the claims and their equivalents are included in the scope of the present invention.

List of Reference Numerals

    100: reaction tank module
    110: bacterial dilution tank
    120: reference reaction tank
    130: sample reaction tank
    140: dilution reaction tank
    200: *Aliivibriofischeri* culture unit
    300: diluted water supply unit
    400: sample supply unit
    500: optical measurement unit
    510: photosensor unit
    600: control unit
    700: analysis unit
    810: casing
    820: monitor unit
    830: waste liquid storage unit
    1000: ecotoxicity monitoring device

**Claims**

1. An ecotoxicity monitoring device (1000) using *Aliivibriofischeri,* comprising:

    a reaction tank module (100) including a reference reaction tank (120), a sample reaction tank

(130), and a dilution reaction tank (140) and provided with a bacterial dilution tank (110) configured to supply a mixed solution including *Aliivibrio fischeri* to the reference reaction tank (120), the sample reaction tank (130), and the dilution reaction tank (140);

an *Aliivibrio fischeri* culture unit (200) configured to supply *Aliivibrio fischeri*, which detect whether a sample is toxic, to the bacterial dilution tank (110);

a diluted water supply unit (300) configured to supply diluted water to the bacterial dilution tank (110), the reference reaction tank (120), and the dilution reaction tank (140);

a sample supply unit (400) configured to supply a sample to the sample reaction tank (130) and the dilution reaction tank (140);

an optical measurement unit (500) configured to measure an amount of luminescence of *Aliivibrio fischeri* in a toxicity assessment substance provided from the reference reaction tank (120) and the sample reaction tank (130) and the dilution reaction tank (140) to which the sample is supplied;

a control unit (600) configured to control the operation of the reaction tank module (100), the *Aliivibriofischeri* culture unit (200), the diluted water supply unit (300), the sample supply unit (400), and the optical measurement unit (500); and

an analysis unit (700) configured to judge whether toxic substances are present in the sample based on the light quantity value provided from the optical measurement unit (600), wherein the analysis unit (700) is configured to calculate a correction coefficient based on the initial light quantity value of the reference reaction tank (120) measured through the optical measurement unit (500) and the final light quantity value measured after a predetermined time has elapsed, and calculate the corrected light quantity values of the sample reaction tank (130) and the dilution reaction tank (140) based on the calculated correction coefficient.

2. The ecotoxicity monitoring device of claim 1, wherein the analysis unit (700) calculates the toxicity index (TIs) of the sample reaction tank, wherein the toxicity index (TIs) of the sample reaction tank is calculated by:

   calculating the correction coefficient of the reference reaction tank (120) according to the following Calculation Formula 1,
   calculating a corrected light quantity value of the sample reaction tank (130) to which the correction coefficient is applied according to the following Calculation Formula 2, and

calculating the toxicity index (TIs) based on the final light quantity value of the sample reaction tank (130) measured after a desired sample has been injected from the sample supply unit (400) into the sample reaction tank (130) and a predetermined time has elapsed according to the following Calculation Formula 3:

[Calculation Formula 1]

$$C0 = \left| \frac{R1}{R0} \right|$$

R0 = Initial light intensity value of mixed solution supplied from bacterial dilution tank (110) to reference reaction tank (120) after 1 minute, and R1 = Final light quantity value after diluted water has been quantitatively injected into mixed solution in reference reaction tank (120) and predetermined time has elapsed;

[Calculation Formula 2]

$$S1 = |S0 \times C0|$$

S0 = Initial light intensity value of mixed solution supplied from bacterial dilution tank (110) to sample reaction tank (130) after 1 minute, and C0 = Correction coefficient;

[Calculation Formula 3]

$$TI_S = \left[ \frac{(S1 - S2)}{S1} \right] \times 100$$

S1 = Corrected light quantity value after initial 1 minute in sample reaction tank to which correction coefficient is applied, and
S2 = Final light quantity value after sample has been quantitatively injected into mixed solution in sample reaction tank (130) predetermined time has elapsed.

3. The ecotoxicity monitoring device of claim 2, wherein the analysis unit (700) calculates the toxicity index (TI$_d$) of the dilution reaction tank (140), wherein the toxicity index (TI$_d$) of the dilution reaction tank (140) is calculated by:

   calculating a corrected light quantity value of the dilution reaction tank (140) to which the correction coefficient of the reference reaction tank (120) is applied according to the following Calculation Formula 4, and
   calculating the toxicity index (TI$_d$) based on the final light quantity value of the dilution reaction tank (140) measured after the sample and diluted water have been injected from the sample supply unit (400) and the diluted water supply unit into the dilution reaction tank (140) at the desired ratio and a predetermined time has elapsed according to the following Calculation Formula 5:

[Calculation Formula 4]

$$D1 = |D0 \times C0|$$

D0 = Initial light intensity value of mixed solution supplied from bacterial dilution tank (110) to dilution reaction tank (140) after 1 minute, and
C0 = Correction coefficient;

[Calculation Formula 5]

$$TI_d = \left[\frac{(D1 - D2)}{D1}\right] \times 100$$

D1 = Corrected light intensity value of dilution reaction tank (140) to which correction coefficient is applied after initial 1 minute, and
D2 = Final light quantity value after sample and diluted water have been quantitatively injected into mixed solution in dilution reaction tank (140) and predetermined time has elapsed.

4. The ecotoxicity monitoring device of claim 3, wherein the analysis unit (700) judges that the calculated correction coefficient is valid if it ranges from 0.6 to 1.3, and judges that a toxic substance is present in the sample if the calculated toxicity index (TIs) of the sample reaction tank (130) ranges from 20 to 80%.

5. The ecotoxicity monitoring device of claim 4, wherein the analysis unit (700) evaluates the toxicity risk of the sample by comparing the toxicity index (TIs) of the sample reaction tank and the toxicity index ($TI_d$) of the dilution reaction tank (140).

6. The ecotoxicity monitoring device of one of claims 3 to 5, wherein the predetermined time taken to measure the final light quantity values of the reference reaction tank, the sample reaction tank, and the dilution reaction tank is 5 minutes.

7. An ecotoxicity monitoring method using *Aliivibrio fischeri,* comprising:

(A) preparing a mixed solution in which *Aliivibriofischeri* and diluted water are mixed in a bacterial dilution tank (110);
(B) supplying the mixed solution in the bacterial dilution tank (110) to each of a reference reaction tank (120), a sample reaction tank (130) and a dilution reaction tank (140);
(C) measuring the initial light quantity value of *Aliivibriofischeri* in the reference reaction tank (120), the sample reaction tank (130), and the dilution reaction tank (140) through an optical measurement unit (500) after a predetermined time has elapsed;
(D) supplying diluted water to the reference reaction tank (120), supplying the sample to the sample reaction tank (130), and supplying predetermined amounts of the sample and diluted water to the dilution reaction tank (140) after the initial light quantity value measurement;
(E) measuring the final light quantity value of *Aliivibriofischeri* in the reference reaction tank (120), the sample reaction tank (130), and the dilution reaction tank (140) through the optical measurement unit (500) after a predetermined time has elapsed; and
(F) providing the initial light quantity values and the final light quantity values of the respective reaction tanks measured through the optical measurement unit (500) to the analysis unit (700) to judge whether a toxic substance is present in the sample at the analysis unit,
wherein the analysis unit (700) is configured to calculate a toxicity index for the toxicity assessment substance in the sample reaction tank (130) and the dilution reaction tank (140) by calculating a correction coefficient based on the initial light quantity value and the final light quantity value of the reference reaction tank (120) measured through the optical measurement unit (500), and calculating the corrected light quantity values of the sample reaction tank (130) and the dilution reaction tank (140) based on the calculated correction coefficient.

1000 — ECOTOXICITY MONITORING DEVICE

100 — REACTION TANK MODULE

110 — BACTERIAL DILUTION TANK

120 — REFERENCE REACTION TANK

130 — SAMPLE REACTION TANK

140 — DILUTION REACTION TANK

200 — *ALIIVIBRIO FISCHERI* CULTURE UNIT

300 — DILUTED WATER SUPPLY UNIT

400 — SAMPLE SUPPLY UNIT

500 — OPTICAL MEASUREMENT UNIT

510 — PHOTOSENSOR UNIT

600 — CONTROL UNIT

700 — ANALYSIS UNIT

**FIG. 1**

**FIG. 2**

**FIG. 3**

1000

510   510   510   500

110

120   130   140

300

400

← — MOVING FLOW OF DILUTED WATER
← --- MOVING FLOW OF SAMPLE

# FIG. 4

PREPARE MIXED SOLUTION IN WHICH *ALIIVIBRIO FISCHERI* AND DILUTED WATER ARE MIXED IN BACTERIAL DILUTION TANK — S100

↓

SUPPLY MIXED SOLUTION OF BACTERIAL DILUTION TANK TO EACH OF REFERENCE REACTION TANK, SAMPLE REACTION TANK AND DILUTION REACTION TANK — S200

↓

MEASURE INITIAL LIGHT QUANTITY VALUE OF *ALIIVIBRIO FISCHERI* IN REFERENCE REACTION TANK, SAMPLE REACTION TANK AND DILUTION REACTION TANK THROUGH OPTICAL MEASUREMENT UNIT AFTER PREDETERMINED TIME HAS ELAPSED — S300

↓

SUPPLY DILUTED WATER TO REFERENCE REACTION TANK, SUPPLY SAMPLE TO SAMPLE REACTION TANK, AND SUPPLY PREDETERMINED AMOUNTS OF SAMPLE AND DILUTED WATER TO DILUTION REACTION TANK AFTER INITIAL LIGHT QUANTITY VALUE MEASUREMENT — S400

↓

MEASURE FINAL LIGHT QUANTITY VALUE OF *ALIIVIBRIO FISCHERI* IN REFERENCE REACTION TANK, SAMPLE REACTION TANK AND DILUTION REACTION TANK THROUGH OPTICAL MEASUREMENT UNIT AFTER PREDETERMINED TIME HAS ELAPSED — S500

↓

PROVIDE INITIAL LIGHT QUANTITY VALUES AND FINAL LIGHT QUANTITY VALUES OF RESPECTIVE REACTION TANKS MEASURED THROUGH OPTICAL MEASUREMENT UNIT TO ANALYSIS UNIT TO JUDGE WHETHER TOXIC SUBSTANCE IS PRESENT IN SAMPLE AT ANALYSIS UNIT — S600

**FIG. 5**

Europäisches
Patentamt
European
Patent Office
Office européen
des brevets

# EUROPEAN SEARCH REPORT

**Application Number**

EP 24 20 5823

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2023/101486 A1 (DONGMOONENT CO LTD [KR]) 8 June 2023 (2023-06-08) * the whole document * ----- | 1,7 | INV.<br>G01N33/18<br>C12Q1/02<br>C12Q1/22 |
| Y | CN 103 940 789 A (LIU XINGHAI) 23 July 2014 (2014-07-23) * the whole document * ----- | 1,7 | G01N21/76 |
| A | CN 111 707 659 A (INST OCEANOGRAPHIC INSTR SHANDONG ACADEMY OF SCIENCES) 25 September 2020 (2020-09-25) * the whole document * ----- | 1-7 | |
| A | US 5 441 873 A (KNIGHT JAN H [GB] ET AL) 15 August 1995 (1995-08-15) * the whole document * ----- | 1-7 | |

**TECHNICAL FIELDS SEARCHED (IPC)**

G01N
C12Q

The present search report has been drawn up for all claims

| Place of search | Date of completion of the search | Examiner |
|---|---|---|
| The Hague | 12 February 2025 | Joyce, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 5823

This annex lists the patent family members relating to the patent documents cited in the above-mentioned European search report.
The members are as contained in the European Patent Office EDP file on
The European Patent Office is in no way liable for these particulars which are merely given for the purpose of information.

12-02-2025

| Patent document cited in search report | | Publication date | Patent family member(s) | | Publication date |
|---|---|---|---|---|---|
| WO 2023101486 | A1 | 08-06-2023 | KR 20230083243 | A | 09-06-2023 |
| | | | WO 2023101486 | A1 | 08-06-2023 |
| CN 103940789 | A | 23-07-2014 | NONE | | |
| CN 111707659 | A | 25-09-2020 | NONE | | |
| US 5441873 | A | 15-08-1995 | AT E175233 | T1 | 15-01-1999 |
| | | | AU 660048 | B2 | 08-06-1995 |
| | | | CA 2118699 | A1 | 18-03-1993 |
| | | | DE 69228064 | T2 | 15-07-1999 |
| | | | EP 0603272 | A1 | 29-06-1994 |
| | | | ES 2128356 | T3 | 16-05-1999 |
| | | | JP 3194046 | B2 | 30-07-2001 |
| | | | JP H06510666 | A | 01-12-1994 |
| | | | US 5441873 | A | 15-08-1995 |
| | | | WO 9305142 | A1 | 18-03-1993 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230136837
- KR 20140093389